# EUROPÄISCHE PATENTANMELDUNG

(11) **EP 0 617 045 A2**
(43) Veröffentlichungstag der Anmeldung: **28.09.1994**
(21) Anmeldenummer: 94103856.4
(22) Anmeldetag: 14.03.1994
(51) Int. Cl.: C07H 15/04

(54) **Verfahren zur kontinuierlichen Herstellung von Alkylglycosiden**

(30) Priorität: 19.03.1993 EP 93104599
(71) Anmelder: Akzo Nobel N.V., NL-6824 BM Arnhem (NL)
(72) Erfinder: Bergfeld, Manfred Josef, Dr., D-63906 Erlenbach (DE); Seifert, Jürgen, Dr., D-63868 Grosswallstadt (DE)
(74) Vertreter: Fett, Günter

(57) **Zusammenfassung**

Es wird ein Verfahren zur Herstellungvon Alkylglycosiden durch kontinuierliche Umsetzung von Monosacchariden mit Fettalkoholen in Gegenwart eines sauren Katalysators beschrieben, das dadurch gekennzeichnet ist, daß man Monosaccharid, Fettalkohol und Katalysator kontinuierlich in einen Rührkessel dosiert, die Umsetzung unter stationären Bedingungen bei einem Umsatz von 97% ± 2,5%, bezogen auf eingesetztes Monosaccharid durchführt, aus dem Rührkessel kontinuierlich Wasser abzieht, und das Reaktionsgemisch in einer Menge aus dem Rührkessel austrägt, welche im wesentlichen der zudosierten Menge an Reaktanden und Katalysatoren entspricht.

## Beschreibung

Die Erfindung betrifft ein kontinuierliches Verfahren zur Herstellung von Alkylglycosiden durch Umsetzung von Monosacchariden mit Fettalkoholen in Gegenwart eines sauren Katalysators.

Alkylglycoside sind bereits seit langem bekannt und werden unter anderem aufgrund ihrer oberflächenaktiven Eigenschaften als Waschmittelrohstoffe verwendet. Hier interessieren insbesondere die Alkylglycoside, deren Alkylrest eine Kohlenstoffzahl von mindestens 8 aufweist.

Da man die Alkylglycoside vollständig aus nachwachsenden Rohstoffen herstellen kann, - sie werden vornehmlich aus Fettalkoholen, die aus natürlichen Fetten erhältlich sind, und Sacchariden gewonnen, - hat es nicht an Bemühungen gefehlt, die industriell einsetzbaren Verfahren zu verbessern. So wurden zahlreiche Katalysatorsysteme entwickelt, um die vorwiegend sauerkatalysierte Reaktion vorteilhaft zu beeinflussen. Es hat auch nicht an Versuchen gefehlt, Verfahren zu entwikkeln, die kontinuierlich durchgeführt werden können.

So gibt es insbesondere eine Reihe von Verfahren, bei denen kontinuierlich C₁-C₄-Glycoside hergestellt werden, die in einem zweiten Schritt mit Fettalkoholen zum eigentlichen waschaktiven Produkt umacetalisiert werden. Diese Verfahren bezeichnet man im allgemeinen als zweistufige Verfahren.

In der US-PS 4,223 129 wird ein kontinuierliches Verfahren beschrieben, bei dem Polysaccharide und entsprechende Monoalkohole in einer Vorrichtung umgesetzt werden, bei der die Hauptreaktion im wesentlichen in einer Rohrschlange stattfindet. Gemäß Patentanspruch 4 dieser US-PS soll das Verfahren auch auf die Umsetzung von Alkylalkoholen mit bis zu 18 Kohlenstoffatomen anwendbar sein. Es hat sich jedoch herausgestellt, daß die Reaktion schwer zu kontrollieren ist, daß die Ausbeuten nicht optimal sind und daß darüber hinaus das bei der Reaktion entstehende Wasser erst zu einem späteren Zeitpunkt abgezogen werden kann, so daß von einem voll kontinuierlichen Verfahren, das zufriedenstellend arbeitet, keine Rede sein kann.

In der europäischen Patentanmeldung Nr. 0 378 710 wird ein Verfahren beschrieben, bei dem ein heterogener saurer Katalysator eingesetzt wird. Wenn das Reaktionsgemisch nicht mehr mit dem Katalysator in Kontakt steht, wird Alkylglycosid durch Zugabe eines apolaren Lösungsmittels auskristallisiert und abgetrennt. Die Mutterlauge wird nach Abtrennen des Lösungsmittels dem Prozeß wieder zurückgeführt.

In der EP-A2-0 448 794 wird ein Verfahren zur Herstellung von Alkylglycosiden beschrieben, bei dem Alkohole mit Sacchariden einstufig umgesetzt werden. Das Verfahren kann auch kontinuierlich betrieben werden. Es ist erforderlich, in Gegenwart eines unpolaren Lösungsmittels, eines Emulgators und verhältnismäßig hoher Mengen eines sauren Katalysators zu arbeiten.

Obwohl bereits eine ganze Reihe von Verfahren zur Herstellung von Alkylglycosiden bekannt sind, besteht ein großes Bedürfnis nach einem verbesserten einstufigen vollkontinuierlichen Verfahren, wobei unter einstufigem Verfahren ein Verfahren zu verstehen ist, bei dem der entsprechende langkettige Alkylalkohol und das Saccharid direkt umgesetzt werden.

Aufgabe der Erfindung ist es deshalb, ein Verfahren zur Verfügung zu stellen, das wirtschaftlich durchzuführen ist, das eine direkte Umsetzung ohne Rückführen von Reaktionsgemisch ermöglicht, das keine weiteren Hilfsstoffe wie Lösungsmittel usw. benötigt, das mit hohen Ausbeuten arbeitet und zu Produkten führt, die sich für den Einsatz als Waschmittelrohstoffe besonders eignen, das flexibel ist und mit dem es möglich ist, die für den jeweiligen Einsatzzweck erforderlichen Eigenschaften wie Viskosität, mittlerer Polymerisationsgrad (DP), Grenzflächenspannung u.dgl. gezielt und reproduzierbar einzustellen. Aufgabe der Erfindung ist es ferner Alkylglycoside zur Verfügung zu stellen, die auch noch bei hohen Feststoffkonzentrationen wie z.B. 70 oder 75% als wässrige Systeme bzw. Lösungen bei Zimmertemperatur pumpbar sind.

Diese Aufgabe wird durch ein Verfahren zur Herstellung von Alkylglycosiden durch kontinuierliche Umsetzung von Monosacchariden mit Fettalkoholen in Gegenwart eines sauren Katalysators gelöst, das dadurch gekennzeichnet ist, daß man Monosaccharid, Fettalkohol und Katalysator kontinuierlich in einen Rührkessel dosiert, die Umsetzung unter stationären Bedingungen bei einem Umsatz von 97% ± 2,5%, bezogen auf eingesetztes Monosaccharid durchführt, aus dem Rührkessel kontinuierlich Wasser abzieht, und das Reaktionsgemisch in einer Menge aus dem Rührkessel austrägt, welche im wesentlichen der zudosierten Menge an Reaktanden und Katalysator entspricht.

Es ist von Vorteil, die Reaktanden und den Katalysator vorher innig zu vermischen und als Gemisch kontinuierlich in den Rührkessel zu dosieren.

Vorzugsweise führt man die Umsetzung bei einem Umsatz von 98 ± 1,5 % durch. Es ist vorteilhaft, die Umsetzung bei einer Temperatur von etwa 80 bis 160°C durchzuführen. Das Wasser wird vorteilhaft in Form von Wasserdampf bei einem Druck von höchstens 1 bar abgezogen. Das molekulare Verhältnis von Monosaccharid zu Fettalkohol beträgt vorzugsweise 1 : 1 bis 1 : 20, insbesondere 1 : 2 - 1 : 6. Als Monosaccharid ist Glucose besonders geeignet. In einer besonders vorteilhaften Ausführungsform des erfindungsgemäßen Verfahrens wird sirupöse Glucose verwendet. Sehr geeignet ist ferner feingmahlene Glucose insbesondere Glucose mit einer Teilchengröße ≦ 30 µm; vorzugsweise wird feingemahlene Glucose mit einem mittleren Teilchendurchmesser von 3 bis 4 µm verwendet.

Das Verfahren wird zweckmäßigerweise homogen katalysiert, d.h. der Katalysator ist homogen im Reaktionsgemisch verteilt.

Es ist vorteilhaft, wenn man durch Entziehen von Wasserdampf die Wasserkonzentration im Rührkessel unter 1 Gew.% hält. Als Katalysatoren sind besonders solche geeignet, welche eine homogene Katalyse gestatten, d.h. der Katalysator ist homogen verteilt bzw. gelöst. Von heterogener Katalyse spricht man z.B. beim Einsatz von Ionenaustauschern als Katalysator.

Als Katalysatoren sind erfindungsgemäß besonders geeignet fluorierte Sulfonsäuren, fluorierte Sulfocarbonsäuren und deren Ester, Sulfocarbonsäureester, wie Sulfocarbonsäuremono-, di- tri- und höhere Ester. Auch Sulfocarbonsäuren sind geeignet. Bei den Estern handelt es sich vor allem um Ester, die sich von Alkoholen mit 1 bis 22 Kohlenstoffatomen ableiten. Vorzugsweise leiten sich die Ester von Alkoholen mit mindestens 8 Kohlenstoffatomen ab, insbesondere von üblichen Fettalkoholen. Neben aliphatischen Sulfocarbonsäuren können auch entsprechende cycloaliphatische aromatische und heterocyclische sowie ungesättigte lineare und cyclische Verbindungen verwendet werden. Anstelle dieser Säuren können auch deren Teil- oder Vollester eingesetzt werden.

Folgende als Beispiele aufgezählte saure Katalysatoren haben sich erfindungsgemäß als vorteilhaft erwiesen. Sulfoessigsäure, Sulfoessigsäuremethylester, Sulfoessigsäuredodecyl/tetradecylester, Sulfobernsteinsäuremonododecylester, Sulfobernsteinsäuredidodecylester/tetradecylester, Sulfobernsteinsäuredimethylester, Sulfopropionsäure, ortho-, meta- oder para-Sulfobenzoesäure und deren Dodecylester, 5-Sulfophthalsäure, 4- oder 5-Sulfoisophthalsäuremono- und di-dodecylester, 4- oder 5-Sulfotrimellithsäure und deren Ester, 4-Sulfo-1,8-naphthalindicarbonsäure und deren Ester.

Neben den Säuren bzw. deren Ester können auch die entsprechenden Anhydride verwendet werden.

Erfindungsgemäß einsetzbare fluorierte Sulfonsäuren sind vor allem die perfluorierten Sulfonsäuren, insbesondere solche aliphatischer und cycloaliphatischer Natur, auch perfluorierte Sulfocarbonsäuren sind hier zu nennen.

Folgende beispielshaft aufgezählten fluorierte Sulfonsäuren haben sich erfindungsgemäß als Katalysatoren als vorteilhaft erwiesen. Trifluormethansulfonsäure, Perfluorethansulfonsäure, Perfluorpropansulfonsäure, Perfluorbutansulfonsäure, Perfluoroctansulfonsäure, Perfluorsulfoessigsäure, Perfluorethylperfluorcyclohexansulfonsäure und deren Mischungen.

Sehr geeignet sind auch Mischungen dieser Katalysatoren untereinander oder mit anderen bekannten Katalysatoren.

In einer weiteren besonders vorteilhaften Ausführungsform des erfindungsgemäßen Verfahrens wird als Katalysator ein Sulfocarbonsäureester verwendet, bei dem der Alkohol, aus dem der Ester aufgebaut ist, der gleiche Fettalkohol ist, der zur Umsetzung als Reaktand eingesetzt wird.

Die Menge des eingesetzten Katalysators oder der Katalysatormischung kann in verhältnismäßig weiten Grenzen variiert werden. Vorzugsweise liegt die Konzentration aber unter 40, insbesondere unter 30 mmol pro Mol eingesetztes Monosaccharid. Ein besonders bevorzugter Bereich ist 1 - 10 mmol/Mol Saccharid.

Der Begriff Alkyl im Sinne der Erfindung bedeutet ein Rest von Fettalkoholen mit vorzugsweise 8 oder mehr Kohlenstoffatomen, der linear oder verzweigt sein kann. Dabei kann es sich bei dem Alkylrest um gesättigte aliphatische Kohlenwasserstoffreste handeln, es kann aber auch ein typischer Rest von Fettalkoholen sein, wie sie aus natürlichen Fetten erhalten werden, so daß außer gesättigten Resten hier auch ungesättigte Reste und deren Gemische zu verstehen sind. Unter Fettalkoholen sind sowohl Alkohole zu verstehen, die aus Naturprodukten zugänglich sind, als auch synthetisch hergestellte Alkanole.

Unter Alkylglycosiden im Rahmen der Erfindung sind Verbindungen zu verstehen, bei denen Alkylreste acetalisch an mono-und/oder oligomeren Zuckerresten (Saccharidresten) gebunden sind.

Stationäre Bedingungen im Rahmen der Erfindung bedeutet, daß die eigentliche kontinuierliche Umsetzung im wesentlichen unter konstanten Bedingungen gefahren wird, wobei im wesentlichen konstant bedeutet innerhalb eines gewissen Toleranzbereiches. So wird der Umsatz, d.h. die Menge des umgesetzten Monosaccharids auf 97% ± 2,5% gehalten. Es versteht sich von selbst, daß durch geeignete Maßnahmen wie schnelleres oder langsameres Zudosieren der Reaktionskomponenten dieser Wert gesteuert wird. Auch die Reaktionstemperatur wird im allgemeinen auf ± 2°C konstant gehalten. Das gleiche gilt sinngemäß im kontinuierlichen Betrieb für die Füllhöhe des Kessels, d.h. im allgemeinen wird eine Schwankungsbreite von ± 2% um den Nennfüllstand eingehalten.

Es versteht sich von selbst, daß vor der eigentlichen kontinuierlichen Umsetzung die Reaktion im Rührkessel angefahren werden muß, bis ein Zustand erreicht ist, der bei der kontinuierlichen Umsetzung stationär gehalten werden soll.

Das Anfahren kann z.B. halbkontinuierlich erfolgen, indem man z.B. den Alkohol vorlegt und zunächst nur Monosaccharid zudosiert, wobei die erforderliche Katalysatorkonzentration z.B. bereits mit dem Alkohol vorgelegt wird.

Es ist auch möglich, in einen leeren Rührkessel das Reaktionsgemisch in den gewünschten Mengenverhältnissen einzudosieren und mit dem Austragen aus dem Kessel erst zu beginnen, wenn eine bestimmte Füllhöhe und der einzustellende Umsatz erreicht ist. Schließlich ist es auch möglich, den Rührkessel zunächst in einer ersten Reaktion ansatzweise zu fahren und nach Erreichen eines Umsatzes von 97 ± 2,5% den Kessel auf kontinuierlichen Betrieb umzuschalten, d.h. die Ausgangssubstanzen kontinuierlich einzudosierenn und kontinuierlich eine entsprechende Menge abzuziehen. Man kann natürlich den Kessel auch mit einer bereits fertigen Reaktionsgemisch füllen, das die den gewählten Reaktionsbedingungen entsprechende stationäre Zusammensetzung aufweist.

Die Ausgangssubstanzen, d.h. Monosaccharid insbesondere Glucose und der Fettalkohol, in dem vorzugsweise der Katalysator vorher gelöst worden ist, werden in den Kessel kontinuierlich dosiert. Bevorzugt werden sie jedoch in einem Mischgefäß unter Verwendung eines Intensivmischers z.B. Intensivkolloidmischer gerührt, so daß eine möglichst homogene Mischung entsteht. Diese wird dann kontinuierlich eindosiert. Die Komponenten können aber auch in -line, d.h. unter Verwendung einer geeigneten Dispergier- und Homogenisierpumpe vermischt und dosiert werden (z.B. eine Supraton-Pumpe der Firma Dorr-Oliver GmbH, Grevenbroich, Deutschland).

Vorzugsweise werden das Gemisch oder die Komponenten von oben in den Kessel dosiert, die entsprechende Menge des umgesetzten Produkts wird an einer entgegengesetzten Stelle kontinuierlich abgezogen.

Es ist möglich, die Glucose als Anhydrit, als Monohydrat oder sogar als wäßrigen Sirup einzusetzen. Die Reaktion im Rührkessel wird durch geeignete Temperaturmeßgeräte gemessen und durch Beheizung oder Kühlung auf entsprechender Temperatur gehalten. Dafür können z.B. Rührkessel mit entsprechendem Doppelmantel und/oder innen- oder außenliegender Wäremaustauscher ausgerüstet sein.

Das Entfernen des Wassers, welches bei der Kondensationsreaktion entsteht bzw. welches als Feuchtigkeit in den Ausgangssubstanzen oder als Bestandteil eines Monohydrats oder der wäßrigen, sirupösen Glucose in das System gelangt, geschieht vorzugsweise durch Anlegen eines geeigneten Vakuums und Einbringen von thermischer Energie, die der benötigten Verdampfungsenthalpie des Wassers entspricht. Es ist zwar möglich, bei normalem Druck bzw. erhöhtem Druck zu arbeiten, vorzugsweise wird jedoch bei einem Druck unter einem bar gearbeitet, vorzugsweise bei Drucken um 20 - 50 m bar.

Die Reaktionstemperatur beträgt im allgemeinen 80 bis 160°C.

Es hat sich als vorteilhaft erwiesen, daß man feste Monosaccharide vor ihrem Einsatz einer Feinmahlung unterwirft, so daß die mittlere Korngröße kleiner als 300 µm insbesondere kleiner als 100 bzw. 30 µm beträgt. In einer besonders vorteilhaften Ausführungsform beträgt die mittlere Korngröße 3 bis 4 µm.

Es war besonders überraschend, daß es mit Hilfe des erfindungsgemäßen Verfahrens möglich ist, ein vollkontinuierliches Verfahren zu realisieren, d.h. die Direktsynthese von Alkylglycosiden ausgehend von Fettalkoholen und Monosacchariden, insbesondere von Glucose. Das entstehende Reaktionsgemisch läßt sich in an sich bekannter Weise einfach aufarbeiten, d.h. in Alkylglycosid und Alkohol trennen. Der Katalysator kann aus dem Gemisch entfernt und rezirkuliert werden bzw. bleibt nach einer Neutralisation als Rückstand übrig. Auch die Aufarbeitung des Reaktionsgemisches kann kontinuierlich erfolgen.

Es war ferner sehr überraschend, daß die nahezu vollständige Saccharidumsetzung in einer Reaktionszeit gelingt, die der von konventionell ansatzweise durchgeführten Verfahren entspricht. Dies ist besonders überraschend, wenn man einen Rührkessel kontinuierlich bei einem Umsatz fährt, der so nahe bei 100% liegt.

Eine weitere Ausführungsform des erfindungsgemäßen Verfahrens besteht darin, daß man anstelle eines Rührkessels zwei oder mehrere hintereinandergeschaltete stationäre Rührkessel verwendet. Dabei wird der letzte Rührkessel stationär bei einem Umsatz von 97 ± 2,5% gefahren. Dies bedeutet, daß jeder Rührkessel für sich betrachtet stationär arbeitet, im Vergleich zueinander in den einzelnen Kesseln aber unterschiedliche Bedingungen wie z.B. unterschiedliche Temperaturen, Katalysatorkonzentrationen, Fettalkohol/Glucose-Verhältnisse, Drucke usw. herrschen können.

Es war weiter überraschend, daß das erfindungsgemäße Verfahren Alkylglycoside liefert, die sich hinsichtlich des Viskositätsverhaltens von üblichen Produkten vorteilhaft unterscheiden.

Deshalb sind ein weiterer Gegenstand der Erfindung Alkylglycoside erhältlich nach einem der vorstehend beschriebenen Verfahren mit einer Viskosität von 50 bis 400 mPas gemessen an einer 10%-igen wässrigen Lösung. Die Viskosität wird mit einem Brookfield Viskosimeter bei Raumtemperatur gemessen, Spindeln 2 und 3; RPM 30 und 60.

Produkte entsprechend dem Stand der Technik weisen Viskositäten von 1300 - 1500 mPas auf.

Die erfindungsgemäßen Alkylgycoside können somit in erheblich höheren Konzentrationen aufgemacht werden und sind bis zu 70 bis 75%-igen wäßrigen Aufmachungen sogar bei Raumtemperatur noch pumpbar. Dies ist bei der Anwendung und beim Transport dieser Produkte von außergewöhnlichem Vorteil. Die Schaumwerte wie Schaumstabilität, Schaumhöhe etc. der Produkte sind gut. Die Grenzflächenspannungswerte und die Solubilisierung von Fetten und Ölen sind hervorragend.

Ein weiterer Vorteil der Erfindung ist darin zu sehen, daß man sirupöse Glucose, wie man sie z.B. durch saure oder enzymatische Hydrolyse von Stärke erhält, direkt mit Fettalkohlen umsetzen kann. Diese sirupösen Glucosen enthalten neben Glucose und Wasser auch noch Oligomere und zu einem geringen Teil Polysaccharide. Sirupöse Glucose ist auch erhältlich aus Saccharose, die man beispielsweise aus Zuckerrüben gewinnt.

Die Erfindung wird durch folgende Beispiele näher erläutert:

### Allgemeine Beschreibung des Verfahrens und der verwendeten Apparatur

Glucose (als Anhydrit, Monohydrat oder wäßriger Sirup) wird mit einer geeigneten Dosiereinheit (Schneckendosierer bei Feststoffen bzw. beheizbare Dosierpumpe bei Sirup) zusammen mit dem Fettalkohol (FA) und dem darin gelösten Katalysator in den entsprechenden, zeitlich konstanten Massenströmen in ein gekühltes 250 ml-Mischgefäß dosiert, welches von einem Intensiv-Kolloidmischer (Ultra Turrax) gerührt wird.

Von diesem Mischgefäß wird ein zeitlich konstanter Massenstrom (der der Summe aus den beiden vorgenannten Teilströmen entspricht) in den kontinuierlichen Rührkesselreaktor (kRk), hier ein 1 L Büchi-Rührreaktor mit Destillationsaufsatz, dosiert.

Der Reaktor ist mit einem Intermig-Rührer, Bodenablaß einer Innentemperaturmessung (Beheizung über Doppelmantel) sowie einem evakuierbaren Auslaß im Deckel ausgerüstet.

Der Reaktor hat unter den angegebenen Betriebsbedingungen und mit den verwendeten Einbauten ein Verweilzeit-Verhalten gezeigt, das zu keinem Zeitpunkt um mehr als 1% vom theoretischen Verlauf abwich.

Das entstehende Reaktionswasser und gegebenenfalls das mit der Glucose eindosierte Wasser wird bei vermindertem Druck kontinuierlich am Kopf des Reaktors abgezogen und über einen Intensivkühler in ein graduiertes Gefäß niedergeschlagen. Die hierbei beobachtete Wasserentstehungsrate wurde als Maß für die Stoffänderungsgeschwindigkeit der eindosierten Glucose (Glc) verwendet (pro Mol eingetragene, wasserfreie Glc entsteht genau 1 Mol Reaktionswasser).

Am Boden des Reaktors wurde das Produkt mit dem entsprechenden, zeitlich konstanten Massenstrom mit Hilfe einer Zahnradpumpe abgezogen, so daß die Füllhöhe des Produktes im Reaktor im Bereich ± 2 mm zu jedem Zeitpunkt konstant war.

Das Endprodukt wurde bei ca. 80°C mit NaOH neutralisiert und dann in an sich bekannter Weise mit einem Dünnfilmverdampfer vom überschüssigen FA befreit.

Die nachfolgenden Versuche sind gemäß der oben beschriebenen Vorgehensweise in dem 1 L-Laborautoklaven gefahren worden. Die mittlere hydrodynamische VWZ (= Volumen der Reaktionsmischung/Volumenstrom der Edukte = τ) ergab sich aus den frei wählbaren (unabhängigen) Variablen
- Temperatur
- FA-Überschuß
- Art und Konzentration der Katalysatoren
- Partikelgrößenverteilung der Glc
zusammen mit der Forderung nach einem bestimmten Glc-Umsatz (z.B. ≧ 99%) als **abhängige**, d.h. nicht frei wählbare Variable.

Nach einer Einlaufzeit von 5 ist im kRk noch ca. 1% des ursprünglich vorgelegten Inhaltes vorhanden. Üblicherweise betrachtet man einen kRk nach dieser Zeit als im Rahmen der Meßgenauigkeit stationär, d.h. der Reaktorinhalt verändert sich danach zeitlich nicht mehr meßbar in Bezug auf Konzentrationen, Dichten, Temperaturen etc.

Es wurden daher für weitergehende und vergleichende Untersuchungen nur solche Produkte verwendet, die aus einem nach obiger Definition stationären kRk erhalten wurden.

### Beispiel 1

Der 1 L Büchi-Raktor wurde mit 500 ml Fettalkohol Lorol S (Henkel), der mit 2 mmol 4-Sulfophthalsäuredilaurylester pro mol FA versetzt war, befüllt. Nach einer Aufheizung des Reaktorinhalts auf 110°C und Anlegen eines Vakuums von 20 mbar wurden in der oben beschriebenen Weise 80.4 g/h Glucose wasserfrei (Cerestar), mit einer mittleren Korngröße von 4 µm, 349,8 g/h Fettalkohol Lorol S, was 4 mol FA/mol Glucose entspricht, sowie 10 mmol 4-Sulfophthalsäuredilaurylester pro Mol Glucose als Katalysator kontinuierlich in den Reaktor eindosiert. Damit wurde der Reaktor als kRk mit einer mittleren hydrodynamischen Verweilzeit τ von 1.0 h betrieben. Die Reaktionstemperatur wurde konstant bei 110°C ± 1.0°C gehalten, das Reaktionsvolumen betrug immer 500 ml ± 10 ml, was durch einen entsprechenden zeitlichen Produktaustrag aus dem kRk (der in der Anfahrphase von ca. 5 h nicht volumenkonstant ist) sichergestellt wurde.

Während der Reaktion wurden bei 20 mbar ≧ 7.9 g/h Wasser aus dem Reaktor abgezogen, was ≧ 99% Glucoseumsatz entspricht. Nach 6 h wurde das nunmehr stationäre Reaktionsprodukt, welches klar und fast weiß war, nach Neutralisation des Katalysators und einer Entfernung des überschüssigen FA auf 1.5% Restgehalt, für die darauffolgenden Analysen verwendet.

Es ergaben sich folgende Werte:

| | |
|---|---|
| freie Glucose | 0.95 Gew.% |
| Oligosaccharidgehalt (DP ≧ 2) | 1.4 Gew.% |
| Monoglucosidgehalt (C₁₂ + C₁₄) | 46.0 Gew.% |
| mittlerer DP des APG-Gemisches | 1.20 |
| Viskosität in 10 Gew.% wäßriger Lösung | 110 mPa.s |

### Beispiel 2

Dieses Beispiel wurde in analoger Weise wie Beispiel 1 gefahren, mit der Ausnahme, daß als Saccharid Glucosemonohydrat (Cerestar) mit dem entsprechenden Massenstrom von 88.4 g/h eindosiert wurde.

Dementsprechend werden bei dieser Reaktion pro Mol umgesetzter Glc.1H₂O zwei Mole Wasser frei.

Aktuell wurden ≧ 15.8 g/h Wasser aus dem Reaktor abgezogen, was ≧ 98% Glucose-Umsatz entspricht.

Nach 6 h wurde, wie in Beispiel 1 beschrieben, ein klares und nahezu weißes Reaktionsprodukt erhalten.

Nach einer Katalysatorneutralisation und einer FA-Abtrennung auf 1.8 Gew.% wurden folgende Analysen am Produkt erhalten:

| | |
|---|---|
| freie Glucose | 1.9 Gew.% |
| Oligosaccharidgehalt (DP ≧ 2) | 1.8 Gew.% |
| Monoglucosidgehalt (C₁₂ + C₁₄) | 48.6% |
| mittlerer DP des APG-Gemisches | 1.25 |
| Viskosität in 10 Gew.% wäßriger Lösung | 275 mPa.s |

### Beispiel 3

Dieses Beispiel wurde in analoger Weise wie Beispiel 1, jedoch mit folgenden Abwandlungen gefahren:
1) als Saccharid wurde ein wäßriger Glucose-Sirup mit 71.4 Gew.% Trockensubstanz und einem DE-Wert (= Dextrose Equivalents) von 99 (Cerestar CX 02660) verwendet.
2) Perfluoroctansulfonsäure wurde als Katalysator in einer Konzentration von 10 mmol/mol Glucose eingesetzt.
3) Die Reaktionstemperatur betrug 120°C
4) Als ursprünglicher Reaktorinhalt beim Start des Versuches wurde ein APG-FA-Katalysatorgemisch vorgelegt, welches in einen Batch-Ansatz aus 80.4 g wasserfreie Glucose, 349.8 g FA Lorol S, 10 mmol Perfluoroctansulfonsäure/mol Glucose als Katalysator bei 120°C mit 99.5% Glucoseumsatz erhalten wurde
5) Die mittlere hydrodynamische Verweilzeit des kRk betrug 90 Minuten
6) Der Reaktor wurde mit 400 ± 10 ml Inhalt betrieben
Die hierfür notwendigen Eduktströme waren wie folgt:

| | |
|---|---|
| Glucose-Sirup: | 60.1 g/h |
| FA Lorol S: | 186.6 g/h |

Es wurden ≧ 21.4 g/h Wasser aus dem Reaktor abgezogen, was einen Glucose-Umsatz von ≧ 98% entspricht.

Das Reaktionsgemisch war zu jedem Zeitpunkt einphasig. Das nach 8 h erhaltene, stationäre Reaktionsprodukt war leicht trüb und von hellbeiger Farbe. Nach einer Neutralisation des Katalysators und der Abtrennung des überschüssigen FA auf 1.9 Gew.% wurden folgende Analysenergebnisse am Produkt erhalten:

| | |
|---|---|
| freie Glucose | 0.4 Gew.% |
| Oligosaccharidgehalt (DP ≧ 2) | 2.8 Gew.% |
| Monoglucosidgehalt (C₁₂ + C₁₄) | 44.0 Gew.% |
| mittlerer DP des APG-Gemisches | 1.25 |
| Viskosität in 10 Gew.% wäßriger Lösung | 125 mPa.s |

Durch die Beispiele 2 und 3 zeigt sich, daß wasserhaltige Saccharide als Reaktions-Ausgangsprodukte vorteilhaft verwendet werden können.

Insbesondere die Verwendung von wäßrigen Sirupen ist hier für eine Direktglucosidierung mit FA vorteilhaft möglich, was nach dem Stand der Technik bisher nicht gelang.

## Patentansprüche

1. Verfahren zur Herstellung von Alkylglycosiden durch kontinuierliche Umsetzung von Monosacchariden mit Fettalkoholen in Gegenwart eines sauren Katalysators, dadurch gekennzeichnet, daß man Monosaccharid, Fettalkohol und Katalysator kontinuierlich in einen Rührkessel dosiert, die Umsetzung unter stationären Bedingungen bei einem Umsatz von 97% ± 2,5%, bezogen auf eingesetztes Monosaccharid durchführt, aus dem Rührkessel kontinuierlich Wasser abzieht, und das Reaktionsgemisch kontinuierlich in einer Menge aus dem Rührkessel austrägt, welche im wesentlichen der zudosierten Menge an Reaktanden und Katalysatoren entspricht.

2. Verfahren nach Anspruch 1, dadurch gekennzeichnet, daß man Monosaccharid, Fettalkohol und Katalysator innig miteinander vermischt und als Gemisch kontinuierlich in den Rührkessel dosiert.

3. Verfahren nach Anspruch 1 oder 2, dadurch gekennzeichnet, daß man die Umsetzung bei einem Umsatz von 98% ± 1,5% durchführt.

4. Verfahren nach einem oder mehreren der Ansprüche 1 bis 3, dadurch gekennzeichnet, daß man die Umsetzung bei einer Temperatur von 80 bis 160°C durchführt.

5. Verfahren nach einem oder mehreren der Ansprüche 1 bis 4, dadurch gekennzeichnet, daß man den Wasserdampf bei einem Druck von höchstens 1 bar abzieht.

6. Verfahren nach einem oder mehreren der Ansprüche 1 bis 5, dadurch gekennzeichnet, daß man durch Entziehen von Wasserdampf die Wasserkonzentration im Rührkessel stets unter 1 Gew.% hält.

7. Verfahren nach einem oder mehreren der Ansprüche 1 bis 6, dadurch gekennzeichnet, daß das molare Verhältnis Monosaccharid zu Fettalkohol 1 : 1 bis 1 : 20 beträgt.

8. Verfahren nach Anspruch 7, dadurch gekennzeichnet, daß das Verhältnis Monosaccharid zu Fettalkohol 1 : 2 bis 1 : 6 beträgt.

9. Verfahren nach einem oder mehreren der Ansprüche 1 bis 8, dadurch gekennzeichnet, daß man als Monosaccharid Glucose verwendet.

10. Verfahren nach Anspruch 9, dadurch gekennzeichnet, daß man sirupöse Glucose verwendet.

11. Verfahren nach Anspruch 9, dadurch gekennzeichnet, daß man feingemahlene Glucose verwendet.

12. Verfahren nach Anspruch 11, dadurch gekennzeichnet, daß man eine Glucose mit einer Teilchengröße < 30 µm verwendet.

13. Verfahren nach Anspruch 11 oder 12, dadurch gekennzeichnet, daß man eine feingemahlene Glucose mit einem mittleren Teilchendurchmesser von 3 bis 4 µm verwendet.

14. Verfahren nach einem der Ansprüche 1 bis 13, dadurch gekennzeichnet, daß man als Katalysator fluorierte Sulfosäuren verwendet.

15. Verfahren nach Anspruch 14, dadurch gekennzeichnet, daß man fluorierte Sulfocarbonsäuren und/oder deren Ester verwendet.

16. Verfahrenn nach einem oder mehreren der Ansprüche 1 bis 13, dadurch gekennzeichnet, daß man als Katalysator Sulfocarbonsäuren oder Sulfocarbonsäureester verwendet.

17. Verfahren nach Anspruch 16, dadurch gekennzeichnet, daß man Sulfocarbonsäureester verwendet, bei denen der Alkohol, aus dem der Ester aufgebaut ist, der gleiche Fettalkohol ist, der bei der Umsetzung als Reaktand verwendet wird.

18. Verfahren nach einem oder mehreren der Ansprüche 1 bis 17, dadurch gekennzeichnet, daß der Katalysator in einer Menge von höchstens 40 mmol/Mol Saccharid verwendet wird.

19. Verfahren nach Anspruch 18, dadurch gekennzeichnet, daß der Katalysator in einer Menge von höchstens 30 mmol/Mol Saccharid verwendet wird.

20. Verfahren nach Anspruch 19, dadurch gekennzeichnet, daß der Katalysator in einer Menge von höchstens 10 mmol/Mol Saccharid verwendet wird.

21. Alkylglycoside, herstellbar nach einem Verfahren gemäß einem oder mehreren der Ansprüche 1 bis 20 mit einer Viskosiät von 50 bis 400 mPa·s, gemessen an einer 10%-igen wässrigen Lösung bei Raumtemperatur.
